Europäisches Patentamt

European Patent Office

Office européen des brevets

(19)

(11) Publication number: **0 266 158**
A2

(12) # EUROPEAN PATENT APPLICATION

(21) Application number: 87309444.5

(22) Date of filing: 26.10.87

(51) Int. Cl.⁴: **B 29 C 61/06**
**A 41 B 13/02**

(30) Priority: 30.10.86 GB 8626007

(43) Date of publication of application:
04.05.88 Bulletin 88/18

(84) Designated Contracting States: **DE FR GB IT SE**

(71) Applicant: ROBINSON & SONS LIMITED
Wheat Bridge
Chesterfield, Derbyshire S40 2AD (GB)

(72) Inventor: Thompson, Ian
Boughton Lane
Clowne Near Chesterfield (GB)

(74) Representative: Goodenough, Nigel et al
A.A. Thornton & Co. Northumberland House 303-306 High
Holborn
London WC1V 7LE (GB)

(54) Composite incorporating elastic material.

(57) A composite, suitable for application to the waist of a
disposable nappy comprises a web of non-woven textile
material impregnated with a stiffening agent e.g. sucrose.
Elastic strands in a stretched condition are adhesively secured
to the web, but the stiffness of the web is sufficient to resist the
contractile forces of the elastic material. The composite is
activated by treatment with a suitable agent, e.g. saturated
steam, in order to reduce the stiffness of the web.

EP 0 266 158 A2

**Description**

## COMPOSITE INCORPORATING ELASTIC MATERIAL

This invention relates to a composite which incorporates elastic material, and more particularly to a composite useful in elasticating articles of wear.

It is well recognized that the fit of certain articles of wear can be improved by incorporating into the articles zones of elastication. It is, however, difficult to provide such zones of elastication at reasonable cost in certain low-cost articles to wear, e.g. disposable nappies for babies. Various techniques have been devised for incorporating elastication at the leg openings of disposable nappies - see for example United States patent 4081301 and British patent application GB 2118021A. Such techniques are satisfactory for the application of elastic running parallel to the machine direction, but cannot be used to apply elastic in a direction perpendicular to the machine direction. Thus, if one of the prior art techniques is used to apply elastication to the leg openings, none of the prior art techniques which require the use of continuous elastic strands can be used to apply elastication to the waist opening.

Further, if discrete lengths of stretched elastic are applied to the waist opening, they will immediately have the effect of contracting the waist opening, and this renders subsequent processing of the disposable nappy extremely difficult.

With a view to avoiding the above mentioned problems and providing a method of elasticating the waist opening of a disposable nappy British patent specification GB 2136678A proposes the use of a web which can be secured to the waist region of a disposable nappy in a substanitally un-tensioned state, and subsequently exposed to heat to cause the web to shrink and assume an elastic state, thereby producing an elastically contractable waistband. A somewhat different technique suitable for the elastication of the wait of a disposable nappy is described in European patent application EP 0151348A. In this case elastic strands in an extended condition are bonded to a substantially in elastic web to produce a composite the stiffness of which decreases significantly when heated, whereby the composite can be applied to the waist area of a disposable nappy in a substantially flat condition, and subsequently exposed to heat in order to produce the desired elastication of the waist area.

Both the technique of GB 2136678 and that of EP 0151348 require the application of heat in order to activate the elastication of the waist area. The present invention provides a composite which can be applied in a flat condition to the waist area of a disposable nappy, and which can subsequently be activated by use of an activating agent rather than by the application of heat. Whilst in the preferred embodiment of the invention the activating agent is steam, and accordingly application of the activating agent will have the effect of raising slightly the temperature of the composite, it is to be understood that the temperature to which the composite must be raised is substantially less than necessary to effect activation by heating as in the case of the prior art, that the heating effect produced in performance of the present invention is subsidiary to the effect of the activating agent, and that the application of heat alone to the composites of the present invention will not produce the desired activation.

According to one aspect of the present invention a composite comprises: an elastic material in a stretched condition; and a carrier to which the elastic material is secured, the carrier being sufficiently resistant to deformation to prevent the elastic material from contracting, wherein the composite is treatable with an activating agent to reduce the resistance of the carrier to deformation to the extent that the carrier no longer prevents contraction of the elastic material.

A preferred embodiment of composite according to the present invention can be manufactured as a flat web and will have little or no tendency to contract elastically in its as manufactured state. Discrete lengths of the composite can accordingly readily be secured to the waist region of a disposable nappy in a direction transverse to the machine direction during manufacture. At a suitable stage in manufacture of the disposable nappy, e.g. after the nappy is cut from the next adjacent nappy and is in the stacker section prior to final packing composite is treated with the desired activating agent with the result that the carrier material can no longer resist the force exerted on it by the stretched elastic material, and the composite together with the backing material of the disposable nappy to which it is secured will contract elastically under the influence of the elastic material.

In the preferred embodiment of the invention the carrier is a web of non-woven material which is impregnated or surface treated with a stiffening agent. The elastic material is preferably in the form of one or elastic strands which are adhesively bonded to the non-woven material. The stiffened web on non-woven material is capable of resisting the force applied to it by the elastic material with the result that the composite remains substantially flat. When treated with an activating agent the stiffening agent is rendered less effective or totally ineffective, whereupon the non-woven web is unable to resist the contracting forces of the elastic material, and the composite accordingly contracts under the influence of the elastic material.

The stiffening agent may be any suitable chemical, and in the preferred embodiment is sucrose. The activating agent is, of course, an agent compatible with the stiffening agent and in the case of the preferred embodiment is saturated steam.

The invention will be better understood from the following description of preferred embodiments thereof, given by way of example only.

In a first embodiment of the invention a carrier of textile material is immersed in a solution of 20% to 40% w/w sucrose in water and is then passed through an expression mangle and dried. Drying may be by any suitable technique, e.g. by means of a non-contact air jet dryer or stenter. Preferably, the finished carrier has a pick-up of sucrose of 200% by dry weight, i.e. 20 grams per square metre (g.s.m.) textile material would pick-up 40 g.s.m. of sucrose by dry weight to give a finished carrier of 60 g.s.m.

The material of the carrier is not critical, and typically any textile material either woven, knitted, or non-woven may be used. For example, a non-woven material of between 12 and 50 g.s.m. of viscose, polyester, polypropylene, or polyethylene, or any combination thereof may be used. Particularly preferred materials would be 20 g.s.m. adhesively bonded polyester, or 20 g.s.m. thermally bonded polypropylene. If desired, a biocide compatible with sucrose may be incorporated in the sucrose solution, or may be applied to the carrier after sucrose impregnation.

After drying, elastic threads or film are secured to the carrier in a stretched condition, e.g. by means of hot melt or pressure sensitive adhesive. The degree of stretch imparted to the elastic material prior to lamination will be determined by the nature of the elastic material and the required characteristics of the finished composite, but may typically be between 60% and 300% extension, with 100% extension being a preferred value. In particularly preferred embodiments of the invention adhesive is laid down on the carrier over an area larger than that covered by the elastic material, and the elastic material is then applied to the carrier with the result that portions of the adhesive material remain exposed. These portions of adhesive material are then used to secure the composite to the backing sheet of a disposable nappy. If it is desired to reel feed the composite the exposed adhesive may be covered with release paper prior to reeling the composite, or the reverse face of the carrier maybe coated with a release coating enabling the composite to be wound on a reel without any interposed release paper.

In use, the composite is fed at right angles to the machine direction and cut into discrete lengths which are bonded by means of suitable adhesive to the surface of the polythene backing of a disposable nappy. At a suitable point in manufacture, e.g. as the nappy is cut and folded, the waistband region of the nappy is treated with jets of saturated steam to reduce the hydrogen bonding of the sucrose to the textile material, thereby reducing the resistance of the textile material to the shirring effect of the elastic, and permitting the composite, and with it the waist region of the nappy, to contract under the influence of the elastic.

It will be appreciated that whilst the invention has been described with particular reference to the the elastication of the waist of a disposable nappy, the composite of the present invention may be used for other elastication purposes, e.g. for the elastication of the leg opening of a disposable nappy, for the elastication of other articles of wear, and indeed for any other use for which a composite which is initially flat and which is subsequently activated is required.

Further, although the invention has been described with particular reference to the use of sucrose as a stiffening agent, it will be appreciated that other stiffening agents may prove appropriate for various applications, and in general any agent which has the required effect, and which can safely be treated with a corresponding activating agent to reduce the resistance of the carrier to the elastic material is suitable.

## Claims

1. A composite comprising: an elastic material in a stretched condition; and a carrier to which the elastic material is secured, the carrier being sufficiently resistant to deformation to prevent the elastic material from contracting, wherein the composite is treatable with an activating agent to reduce the resistance of the carrier to deformation to the extent that the carrier no longer prevents contraction of the elastic material.

2. A composite according to claim 1 wherein the carrier is of textile material.

3. A composite according to claim 2 wherein the carrier is a non-woven material.

4. A composite according to any preceding claim wherein the elastic material comprises one or more elastic strands secured to the surface of the carrier

5. A composite according to claim 4 wherein the or each elastic strand is adhesively bonded to the carrier.

6. A composite according to claim 5 wherein the adhesive extends over a larger area of the surface of the carrier than the area covered by the elastic material whereby portions of adhesive are exposed and may be utilized to secure the composite to an article to be elasticated.

7. A composite according to any preceding claim wherein the composite is impregnated or surface treated with a stiffening agent which is rendered less effective or ineffective by the activating agent.

8. A composite according to claim 7 wherein the stiffening agent is sucrose.

9. A composite according to any preceding claim wherein the activating agent is steam, preferably saturated steam.

10. A method of elasticating the waist and/or leg openings of a disposable nappy, the method comprising adhesively securing a length of composite material according to any preceding claim to the backing material of the nappy and activating the composite as the nappy is cut from the following nappy and folded.